# EUROPEAN PATENT SPECIFICATION

(11) **EP 1 979 734 B1**
(45) Date of publication and mention of the grant of the patent: **09.05.2018**
(21) Application number: 06728509.8
(22) Date of filing: 24.03.2006
(51) Int. Cl.: G01N 21/25, G01N 21/47, G01N 33/02, G01N 21/84

(54) **PORTABLE DEVICE FOR THE QUALITY CONTROL OF PRODUCTS**
TRAGBARE VORRICHTUNG ZUR QUALITÄTSKONTROLLE VON PRODUKTEN
DISPOSITIF PORTABLE PERMETTANT DE CONTROLER LA QUALITE DE PRODUITS

(30) Priority: 01.02.2006 IT RE20060010
(43) Date of publication of application: 15.10.2008
(73) Proprietor: SACMI Cooperativa Meccanici Imola Società Cooperativa, 40026 Imola (Bologna) (IT)
(72) Inventor: ZAULI, Bruno, I-40026 IMOLA (Bologna) (IT); REMONDINI, Marco, I-40026 IMOLA (BOLOGNA) (IT); BOSI, Gildo, I-40026 IMOLA (Bologna) (IT)
(74) Representative: Corradini, Cesare
(86) International application number: PCT/IT2006/000190
(87) International publication number: WO 2007/088563

(56) References cited:
- DE-A1- 3 840 078
- JP-A- 8 292 147
- JP-A- 2002 139 442
- JP-A- 2004 226 357
- US-A- 4 660 984
- US-A1- 2004 149 916
- US-B1- 6 512 577
- PATENT ABSTRACTS OF JAPAN vol. 2000, no. 22, 9 March 2001 (2001-03-09) -& JP 2001 133394 A (KUBOTA CORP), 18 May 2001 (2001-05-18)
- PATENT ABSTRACTS OF JAPAN vol. 011, no. 216 (P-595), 14 July 1987 (1987-07-14) -& JP 62 034035 A (NIPPON TECTRON CO LTD), 14 February 1987 (1987-02-14)
- PATENT ABSTRACTS OF JAPAN vol. 2000, no. 06, 22 September 2000 (2000-09-22) -& JP 2000 088747 A (TAKAYA CORP), 31 March 2000 (2000-03-31)

## Description

### TECHNICAL FIELD

The present invention relates to the technical field of the quality control of products in particular, such as fruit and vegetables, and of other products with similar features. In particular, the present invention relates to the quality control by spectroscopic analysis of the product.

### BACKGROUND ART

Devices have been setup, which substantially comprise a chain conveyer whose links consist of bowls, each adapted for receiving a fruit, associated to spectroscopic analysis means.

According to the prior art, the spectroscopic analysis means comprises means for lighting the fruit and means for gathering the light refracted by the fruit and for the spectroscopic analysis of the same.

The chain moves with a continuous motion in front of said lighting means and the light gathering and spectroscopic analysis means is arranged so that upon the passage of the fruit, at least a portion of the light crossing the fruit is gathered and sent to a spectrometer.

The data gathered by the spectrometer are analysed by a processor and are converted into parameters telling the ripening level and in general, the product quality.

An equipment of the above type is described, for example, in document EP 1215480 A1.

The fixed installations of the type mentioned above, besides a high cost and the poor versatility, exhibit some disadvantages that restrict the use thereof.

They comprise lighting devices of sensible power, in the range of 2000 W, which are necessary for making the measurement as little as possible influenced by the ambient lighting, but they create problems of undesired product heating in the case of stoppage of the conveyer chain of the same.

The problem has been solved by providing intermitted lighting means, but the high power of the lighting means requires a strict control, which at each measurement should ensure the achievement of steady conditions and therefore the reliability and repeatability of the measurements made.

Moreover, the conveyer links are designed for an average product size, and are not much suitable for the control of products whose size considerably differs from the average; to this end, we may consider kiwis and melons.

To obviate the above disadvantages, portable devices have also been devised, which substantially comprise a body that can be gripped like a gun, which exhibits an appendix at the end of which there is a crown of lighting means at the centre of which there is arranged an objective for gathering and concentrating the refracted light.

The use of such portable devices provides for the appendix end to rest on the product surface so that the refracted light is gathered by the objective.

Also the known portable devices, however, have proven partly unsatisfactory, since the intermittent actuation of the lighting means does not guarantee that they are always at steady state when the measurements are made; moreover, the poor lighting power makes the device very sensitive to the influence of the ambient lighting.

Last but not least, the concentration of lighting in limited product zones makes the device unsuitable for controlling fruits of a size larger than the average and with a hard skin. US 6, 512, 577 B1 discloses an apparatus and method for measuring and correlating characteristics of fruit with visible/near infra-red spectrum. The apparatus has at least two light sources having different wavelengths, one in the range 250-499 nm and the other in the range 500-1150 nm. Light refracted of the two spectra directed to an analyzed by a spectrometer in order to deduce the properties of the fruit. The device maybe portable. The lamp is held at a resting voltage of 2-Volts. When a measurement is taken, the lamp is ramped up to the desired voltage, a brief delay allows the lamp output to stabilize, then spectra are acquired. After data acquisition, the lamp is ramped down to the resting voltage.

The object of the present invention is to provide a unit for the spectroscopic control of products, preferably vegetal products, which should be of such a size as to be contained in a case while not exhibiting the disadvantages of the prior art.

### DISCLOSURE OF INVENTION

Said object is achieved by a device, having the features described in the claims.

In particular, the device according to the invention comprises '*inter alia'* the following :
arranging a circular crown of at least two lamps, Preferably angularly spaced from one another, about means adapted for resting on the product to be analysed;
arranging a spectrometer below the product, powering said lamps at a reduced voltage as compared to the nominal power supply voltage so as to keep them pre-heated in a stand-by status,
changing the power supply voltage in an instant manner up to the nominal power supply voltage, for causing the emission of a light, flash for the time required to carry out the measurement.

The advantages and the functional and construction features of the device according to the invention will appear clearly from the following description which, with reference to the annexed drawings, illustrates a particular preferred embodiment thereof, made by way of a nonlimiting example. The broadest scope of the invention is limited as defined by appended independent claim 1.

### BRIEF DESCRIPTION OF DRAWINGS

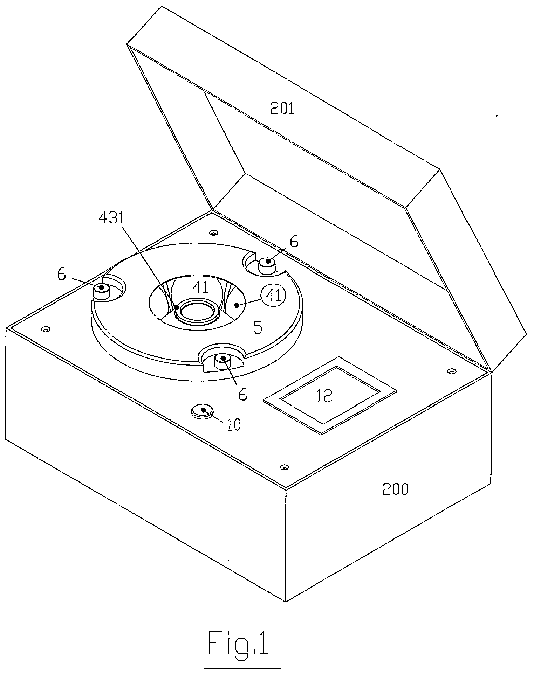

Fig. 1 shows a perspective view of the exemplary device;
Fig. 2 shows a plan view of the exemplary device;
Fig. 3 shows a plan view of the exemplary device without a detail;
Fig. 4 shows section IV-IV marked in Fig. 2, with the electrical block diagram.
Fig. 5 shows section IV-IV marked in Fig. 2, wherein there is shown a variation of the electrical operating diagram.
Fig. 6 shows a second embodiment of the exemplary device, in the same section as in Fig. 5.

### BEST MODE FOR CARRYING OUT THE INVENTION

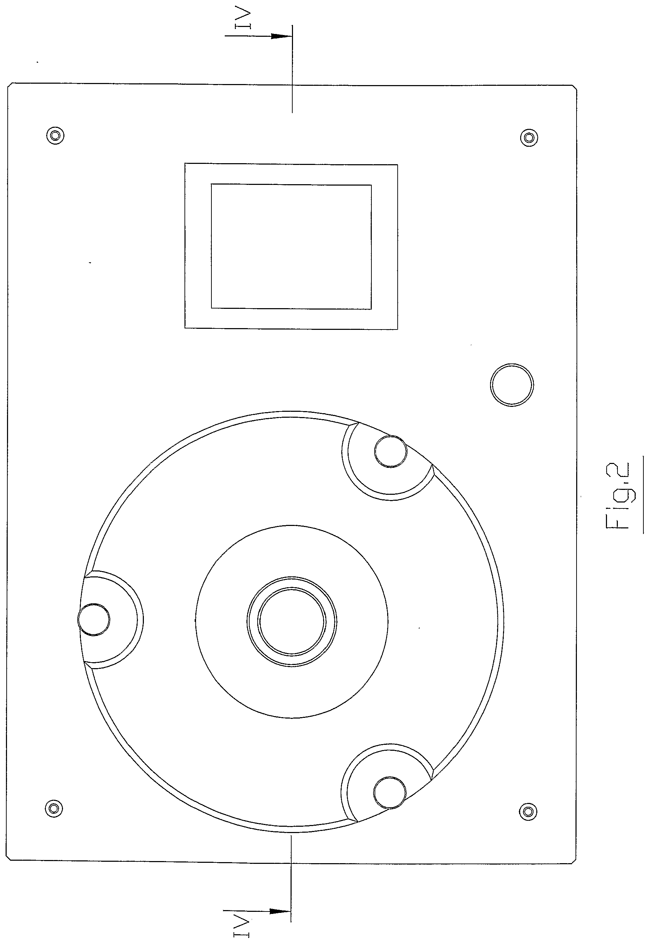

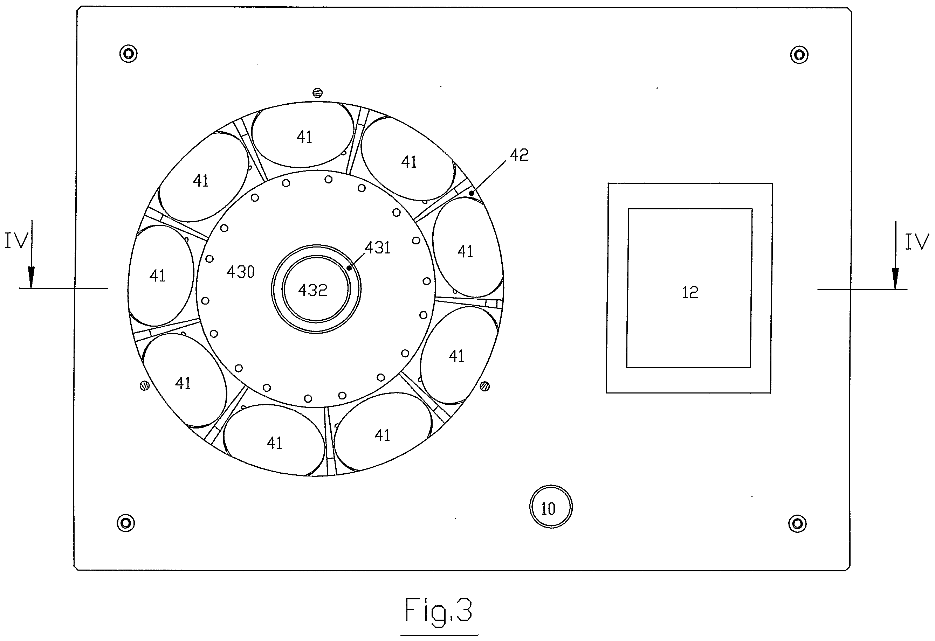

In the figures from Fig. 2 to Fig. 4, it can be seen that the exemplary device comprises a bottom plate 1 and a top plate 2 connected by four posts 3 with adjustable length.

In particular, each post comprises a portion 31 fixed to plate 1 on which a portion 32 turnable and coupled to plate 2 is screwed.

On portion 32 there is arranged a toothed pulley 33, and pulleys 33 of each post are connected by a toothed belt 34, so as to rotate by identical amounts for allowing the adjustment of the distance between plates 1 and 2 keeping them strictly parallel to each other.

Plate 2 comprises a hole 21 of a diameter equal to that of an underlying crown 4 of lighting means.

In the example shown, crown 4 consists of nine lamps 41 supported by a truncated-cone sheet bell 42 so as to be converging upwards with an angle of about 45° relative to the vertical.

Bell 42 is supported by a hollow central body 43 whose top truncated-cone surface 430 is inclined by 45° relative to the vertical and is mirror polished, so as to be reflecting.

The hollow truncated-cone body 43 is supported by a bottom shelf 44 connected to the bottom plate 1, to which there is also fixed a spectrometer 45 of known construction whose lens for receiving the light beams to be analysed is arranged below a hole 440 of the shelf aligned with the cavity 432 of the hollow body.

Cavity 432 comprises an edge 431 at the top adapted for receiving the fruit to be analysed by rest. In the exemplary embodiment shown, said edge is of deformable type, but it may also be of the stiff type, according to the type of product to be analysed.

Into cavity 432 there is arranged a lens 433 adapted for concentrating the light beam that has crossed the fruit on the input lens of the underlying spectrometer 45. Hole 21 is partly closed by a top body 5 having a hole 51 coaxial with hole 21.

Hole 51 has a diameter of intermediate value between that of hole 21 and that of cavity 432, and is arranged at the top of a specular or reflecting truncated-cone surface 52 inclined by an amount similar to the amount of the inclination of the truncated-cone surface 430 relative to the vertical.

It is noted that in other exemplary embodiments, the top body 5 is fixed to the top plate 2 by three equally-spaced screws 6 arranged on the same circumference.

Lamps 41, which in the example shown are of the halogen type with back aluminium parabola, normally available on the market, address a light beam shaped as circular crown against the product, not shown, resting on the deformable edge 431; the width of the lighted zone is adjusted by varying the height of plate 3 by the actuation of pulleys 33.

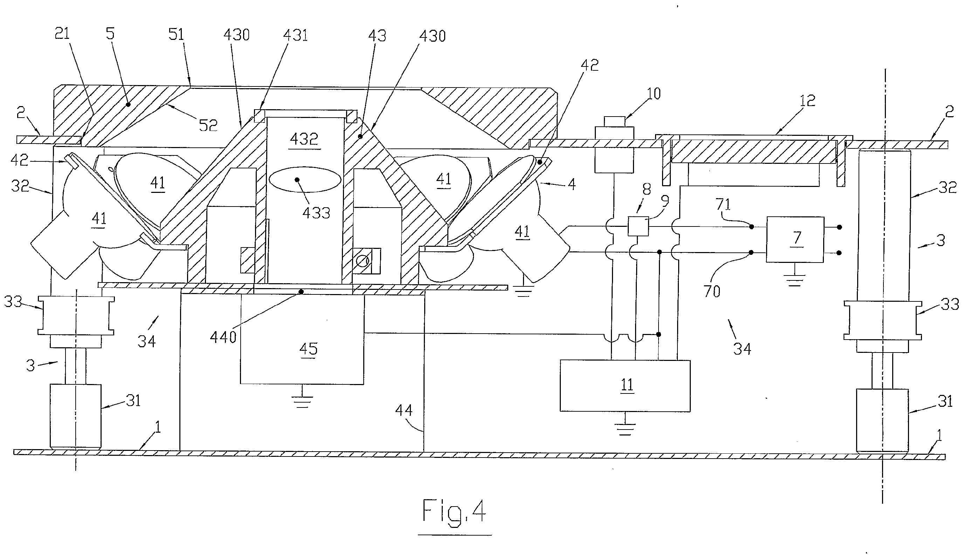

The maximum power absorbed by the nine lamps as a whole is in the range of 200 W, and they are powered by the circuit shown in blocks in Fig. 4.

The circuit comprises an electronic or stabilised power supply 7, having two output terminals, of which one 70 at reduced voltage and one 71 at nominal voltage. The stabiliser input is intended for being connected to an energy source, such as the 220 VAC mains.

In the exemplary embodiment shown, the voltage available at the output terminal 70 is equal to about one fifth the nominal voltage available at the output terminal 71, whose value is 24 Volts.

The output terminal 70 of the power supply 7 is intended for powering lamps 41, the spectrometer 45, a microprocessor 11, and display means at reduced voltage. Lamps 41 are also directly connected to output 71, at nominal voltage, of the power supply 7 through a power circuit 8, whose actuation is commanded and controlled by the microprocessor 11.

The power circuit (Fig. 4) comprises a controlled switch 9, which can be obtained with any type of switching device suitable for the purpose, for example with a power transistor type IGBT, or GTO, or MOS/FET or SCR.

An external button 10 sends an actuation signal to microprocessor 11 which controls the actuation of the power circuit, that is, with reference to the circuit of Fig. 4, the conduction of the switching device for a very short time interval, in the range of one second. In this way, lamps 41 are powered at the nominal voltage, which causes the emission of a pulse, or flash, of light sufficient for making the measurement.

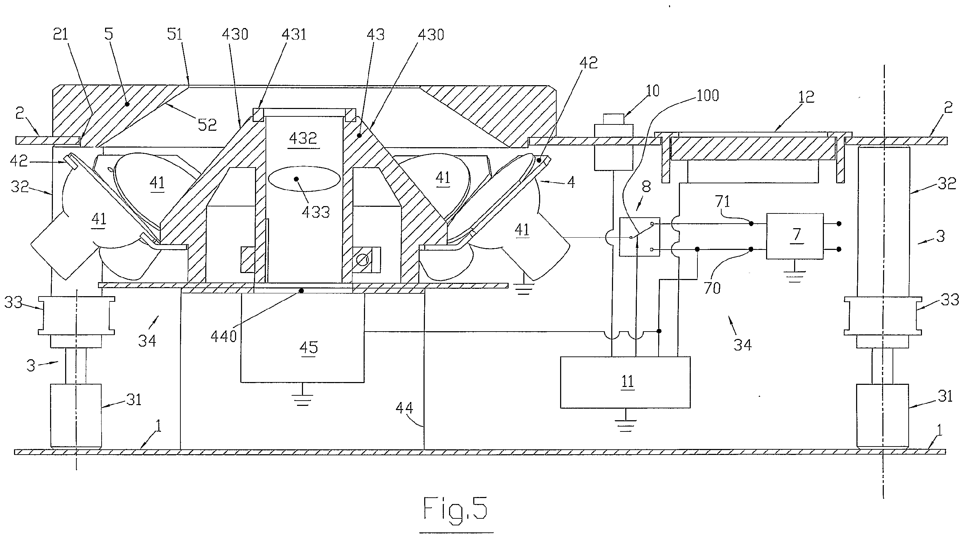

According to the variation of Fig. 5, the controlled switch comprises a usual commutator switch 100 adapted for alternately connecting the lamps to terminal 70 (reduced voltage output) or 71 (nominal voltage output). In the case of the circuit of Fig. 5, microprocessor 11, following the actuation signal generated by the pressure of button 10, controls the switching of the commutator switch 100 from terminal 70 to terminal 71, so as to power the lamps at the nominal voltage rather than the reduced voltage. After a very short time interval, sufficient for the emission of the light pulse by the lamps, microprocessor 11 controls the switching of the commutator switch 100 again from terminal 71 to terminal 70 so as to power the lamps at the reduced voltage again. From the description above it is clear that normally, lamps 41 are powered at a reduced voltage, which allows keeping them pre-heated in a stand-by status. When the measurement must be made, the operator presses button 10, thus generating an actuation signal received by microprocessor 11.

Following the actuation signal, microprocessor 11 controls the actuation of the power circuit which powers the lamps at the nominal voltage for a very short time interval, substantially instantaneous. The quick variation of the power supply voltage causes the emission of the pulse, or flash, of light required to make the measurement.

This has the advantage that the generation of the light pulse occurs very quickly so that the product is subject to the temperature emitted by the lamps, harmful for the storage thereof, only for the minimum time required to make the measurement.

Microprocessor 11 is also connected to the display means adapted for displaying the results of the measurement made, such as a screen 12.

Button 10 may be replaced by a device associated to the deformable edge 431, for the automatic transmission of the signal generated by the rest of the product to the microprocessor.

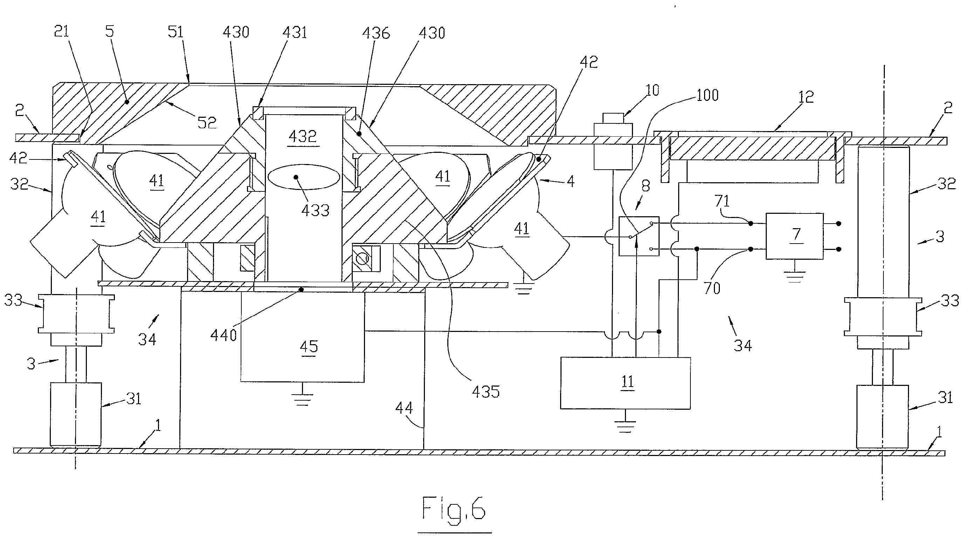

Fig. 6 shows a further exemplary embodiment relating to the support means of the fruit to be analysed.

In Fig. 6, the components in common with Fig. 5 are indicated with the same reference numerals.

The exemplary embodiment of Fig. 6 has the task of allowing the analysis of fruits having sizes very different from one another.

To this end, the hollow truncated-cone body 43 consists of a fixed bottom portion 435 fixed to the bottom shelf 44 connected to the bottom plate 1, and of a removable top portion 436 which carries the usual edge 431.

In the example shown, lens 433 is contained in the removable top portion, but the same may be more conveniently arranged in the fixed bottom portion.

The removable portion 436 is screwed on the fixed portion, and is available in different sizes, specifically different heights, based on the diameter of the fruit to be analysed.

The larger the fruit diameter, the higher the height of the removable portion.

For very small fruits, the use of a removable portion exhibiting the top seat defined by edge 431 of conveniently smaller diameter is provided.

The device now described is contained in a case 200 comprising a cover 201, whose size in the range of 300 x 400 x 200 mm make the whole easy to carry.

The device operation is as follows.

The device is connected to the mains, and lamps 41 are powered in stand-by condition at low voltage until they reach a temperature close to the steady temperature.

The height of the top plate 2 is adjusted based on the fruit diameter for concentrating the light beam on the fruit compatibly and suitably for the size of the fruit itself.

The fruit is rested on edge 431 which masks the ambient light, and then button 10 is actuated.

A light flash is thus emitted at the maximum power of the lamps, and for the instant required for making the measurement. The measurement results are displayed on the screen, and the operation can be repeated indefinitely without the need of waiting that lamps 41 reach the steady conditions again, which are maintained by the low voltage power supply.

It is noted that in place of microprocessor 11 it is also possible to use a microcontroller or, more in general, any programmable logic device. Moreover, the microprocessor may be provided with means adapted for interfacing it to data transmission networks or to other processors.

## Claims

1. A portable device for the quality control of products comprising a power supply (7), a circular crown (4) of lighting lamps (41) arranged to address a light beam shaped as a circular crown against a product to be inspected, powered by said power supply (7), a spectrometer (45) having a receiving objective, the receiving objective being arranged at the centre of said circular crown (4) of lighting lamps (41), said spectrometer (45) being provided with means (431) adapted for resting on the product to be checked, a microprocessor (11), display means, said spectrometer (45) being associated to said microprocessor (11) which provides the measurement data on said display means, a power circuit (8) whose actuation is commanded and controlled by the microprocessor (11) in response to an actuation signal, wherein said power supply (7) is arranged for powering the lamps (41) of said circular crown (4) permanently, and in stand-by, at a reduced voltage, lower than the nominal voltage delivered by the power supply, and instantaneously at the nominal voltage supplied by the power supply (7) through the power circuit (8) actuated in response to said actuation signal,
- wherein said portable device further comprises a centrally hollow body (43) having a base, a truncated-cone reflecting wall (430) adapted to support said circular crown (4) of lighting lamps (41),
- wherein the centrally hollow body (43) comprises a cylindrical cavity (432) having at its top said means (431) adapted for resting on the product to be checked, and having arranged at its base the receiving objective of said spectrometer (45), a top body (5), arranged above the centrally hollow body (43), provided with a hole (51) coaxial with the cylindrical cavity (432) of the centrally hollow body (43) and having an intermediate diameter between that of the cylindrical cavity (432) of the centrally hollow body (43) and that of the circular crown (4) of lighting lamps (41),
- wherein the surface (52) of the top body (5) facing the centrally hollow body (43) facing the truncated-cone reflecting wall (430) of the same has a truncated cone shape and is reflecting.

2. A device according to claim 1, **characterised in that** said power circuit comprises a controlled switch (9).

3. A device according to claim 2, **characterised in that** said controlled switch (9) is a commutator (100).

4. A device according to claim 2, **characterised in that** said controlled switch (9) is a power transistor.

5. A device according to claim 1, **characterised in that** the centrally hollow body (43) comprises a fixed bottom portion (435) and a removable top portion (436) whose sizes are selected based on the size of the product to be analysed.

6. A device according to claim 1, **characterised in that** said means comprises a ring (431) intended for receiving the product.

7. A device according to claim 6, **characterised in that** said ring (431) is deformable.

8. A device according to claim 1, **characterised in that** into the cylindrical cavity (432) of the hollow body (43) there is arranged a lens (433) for concentrating the light beam refracted by the product on the spectrometer objective.

9. A device according to claim 1, **characterised in that** the distance between the hollow body (43) and the top body (5) is adjustable.

10. A device according to claim 9, **characterised in that** the hollow body (43) is integral to a bottom plate (1) and the top body (5) is integral to a top plate (2), said plates being connected by posts (3) of adjustable length.

11. A device according to claim 10, **characterised in that** said posts (3) comprise a first portion (31) fixed to the bottom plate (1) and a second portion (32), screwable on the first one, associated to the top plate (2), the second portions of the posts (3) being constrained to rotate by the same amount.

12. A device according to claim 1, **characterised in that** said display means comprises a screen (12).

## Patentansprüche

1. Transportables Gerät zur Qualitätskontrolle von Produkten, umfassend eine Energiezufuhr (7), eine kreisförmige Krone (4) aus Beleuchtungslampen (41), die dafür angeordnet sind, einen Lichtstrahl, der zu einer kreisförmigen Krone geformt ist, auf ein zu inspizierendes Produkt zu richten, durch die Energiezufuhr mit Energie versorgt, ein Spektrometer (45) mit einem aufnehmenden Objektiv, wobei das aufnehmende Objektiv in der Mitte der kreisförmigen Krone (4) aus Beleuchtungslampen (41) angeordnet ist, wobei das Spektrometer (45) mit Mitteln (431) ausgestattet ist, die dafür eingerichtet sind, auf dem zu prüfenden Produkt zu ruhen, einen Mikroprozessor (11), Anzeigemittel, wobei das Spektrometer (45) dem Mikroprozessor (11) zugeordnet ist, der die Messdaten auf dem Anzeigemittel bereitstellt, einen Leistungskreis (8), dessen Betätigung durch den Mikroprozessor (11) in Reaktion auf ein Betätigungssignal angewiesen und gesteuert wird, wobei die Energieversorgung (7) dafür angeordnet ist, die Lampen (41) der kreisförmigen Krone (4) dauerhaft mit Energie zu versorgen und im Bereitschaftsmodus mit verminderter Spannung, die niedriger als die von der Energieversorgung gelieferte Sollspannung ist, und sofort mit der Sollspannung, die von der Energiezufuhr (7) zugeführt wird, über den Leistungskreis (8), der in Reaktion auf das Betätigungssignal betätigt wird,
wobei das transportable Gerät ferner einen mittig hohlen Körper (43) umfasst, mit einer Grundfläche, einer kegelstumpfförmigen reflektierenden Wand (430), die dafür eingerichtet ist, die kreisförmige Krone (4) aus Beleuchtungslampen (41) zu tragen,
wobei der mittig hohle Körper (43) einen zylinderförmigen Hohlraum (432) umfasst, der an seiner Oberseite die Mittel (431) aufweist, die dafür eingerichtet sind, auf dem zu prüfenden Produkt zu ruhen, und angeordnet an seiner Grundfläche das aufnehmende Objektiv des Spektrometers (45), einen oberen Körper (5), der über dem mittig hohlen Körper (43) angeordnet ist, mit einer Öffnung (51) koaxial mit dem zylinderförmigen Hohlraum (432) des mittig hohlen Körpers (43) versehen ist und einen mittleren Durchmesser zwischen dem des zylinderförmigen Hohlraums (432) des mittig hohlen Körpers (43) und dem der kreisförmigen Krone (4) aus Beleuchtungslampen (41) aufweist, wobei die Oberfläche (52) des oberen Körpers (5), die zum mittleren hohlen Körper (43) weist, der zur kegelstumpfförmigen reflektierenden Wand (430) desselben weist, eine Kegelstumpfform aufweist und reflektierend ist.

2. Gerät nach Anspruch 1, **dadurch gekennzeichnet, dass** der Leistungskreis einen gesteuerten Schalter (9) umfasst.

3. Gerät nach Anspruch 2, **dadurch gekennzeichnet, dass** der gesteuerte Schalter (9) ein Kommutator (100) ist.

4. Gerät nach Anspruch 2, **dadurch gekennzeichnet, dass** der gesteuerte Schalter (9) ein Leistungstransistor ist.

5. Gerät nach Anspruch 1, **dadurch gekennzeichnet, dass** der mittig hohle Körper (43) einen feststehenden Bodenabschnitt (435) und einen abnehmbaren oberen Abschnitt (436) umfasst, deren Größen basierend auf der Größe des zu analysierenden Produkts gewählt sind.

6. Gerät nach Anspruch 1, **dadurch gekennzeichnet, dass** die Mittel einen Ring (431) umfassen, der zur Aufnahme des Produkts vorgesehen ist.

7. Gerät nach Anspruch 6, **dadurch gekennzeichnet, dass** der Ring (431) verformbar ist.

8. Gerät nach Anspruch 1, **dadurch gekennzeichnet, dass** im zylinderförmigen Hohlraum (432) des hohlen Körpers (43) eine Linse (433) zum Bündeln des Lichtstrahls angeordnet ist, der vom Produkt auf das Spektrometerobjektiv gebrochen wird.

9. Gerät nach Anspruch 1, **dadurch gekennzeichnet, dass** der Abstand zwischen dem hohlen Körper (43) und dem oberen Körper (5) einstellbar ist.

10. Gerät nach Anspruch 9, **dadurch gekennzeichnet, dass** der hohle Körper (43) integraler Bestandteil einer unteren Platte (1) ist und der obere Körper (5) integraler Bestandteil einer oberen Platte (2) ist, wobei die Platten durch Säulen (3) mit einstellbarer Länge verbunden sind.

11. Gerät nach Anspruch 10, **dadurch gekennzeichnet, dass** die Säulen (3) einen ersten Abschnitt (31), der an der unteren Platte (1) befestigt ist, und einen zweiten Abschnitt (32) umfassen, der auf den ersten Abschnitt geschraubt werden kann und der oberen Platte (2) zugeordnet ist, wobei die zweiten Abschnitte der Säulen (3) darauf beschränkt sind, sich in gleichem Umfang zu drehen.

12. Gerät nach Anspruch 1, **dadurch gekennzeichnet, dass** die Anzeigemittel einen Bildschirm (12) umfassen.

## Revendications

1. Dispositif portable pour le contrôle qualité de produits comprenant une source d'alimentation (7), une couronne circulaire (4) de lampes d'éclairage (41) disposées pour diriger un faisceau lumineux de la forme d'une couronne circulaire sur un produit à inspecter, alimenté par ladite source d'alimentation (7), un spectromètre (45) disposant d'un objectif de réception, l'objectif de réception étant disposé au centre de ladite couronne circulaire (4) de lampes d'éclairage (41), ledit spectromètre (45) étant doté de moyens (431) conçus pour
reposer sur le produit à vérifier, un microprocesseur (11), moyen d'affichage, ledit spectromètre (45) étant associé audit microprocesseur (11) qui fournit les données de mesure sur ledit moyen d'affichage, un circuit d'alimentation (8) dont l'actionnement est commandé et contrôlé par le microprocesseur (11) en réponse à un signal d'actionnement, dans lequel
ladite source d'alimentation (7) est disposée pour alimenter les lampes (41) de ladite couronne circulaire (4) en permanence, et en veille, à une tension réduite, inférieure à la tension nominale délivrée par la source d'alimentation, et instantanément à la tension nominale fournie par la source d'alimentation (7) par le biais du circuit d'alimentation (8) actionné en réponse audit signal d'actionnement,
dans lequel
ledit dispositif portable comprend également un corps creux en son centre (43) disposant d'une base, une paroi réfléchissante en forme de cône tronqué (430) conçue pour supporter ladite couronne circulaire (4) de lampes d'éclairage (41),
dans lequel
le corps creux en son centre (43) comprend une cavité cylindrique (432) possédant sur sa partie supérieure lesdits moyens (431)
conçus pour reposer sur le produit à vérifier et ayant l'objectif de réception dudit spectromètre (45) disposé sur sa base, un corps supérieur (5), disposé au-dessus du corps creux en son centre (43), doté d'un orifice (51) coaxial à la cavité cylindrique (432) du corps creux en son centre (43) et disposant d'un diamètre intermédiaire situé entre celui de la cavité cylindrique (432) du corps creux en son centre (43) et celui de la couronne circulaire (4) de lampes d'éclairage (41), dans lequel
la surface (52) du corps supérieur (5) orientée vers le corps creux en son centre (43) orienté vers la paroi réfléchissante en forme de cône tronqué (430) de ce dernier dispose d'une forme de cône tronqué et est réfléchissante.

2. Dispositif selon la revendication 1, **caractérisé en ce que** ledit circuit d'alimentation comprend un interrupteur commandé (9).

3. Dispositif selon la revendication 2, **caractérisé en ce que** ledit interrupteur commandé (9) est un commutateur (100).

4. Dispositif selon la revendication 2, **caractérisé en ce que** ledit interrupteur commandé (9) est un transistor de puissance.

5. Dispositif selon la revendication 1, **caractérisé en ce que**
le corps creux en son centre (43) comprend une partie inférieure fixe (435) et une partie supérieure amovible (436) dont les tailles sont choisies en fonction de la taille du produit à analyser.

6. Dispositif selon la revendication 1, **caractérisé en ce que**
ledit moyen comprend une bague (431) destinée à recevoir le produit.

7. Dispositif selon la revendication 6,
**caractérisé en ce que** ladite bague (431) est déformable.

8. Dispositif selon la revendication 1, **caractérisé en ce que**
dans la cavité cylindrique (432) du corps creux (43) une lentille (433) est placée pour concentrer le faisceau lumineux réfracté par le produit sur l'objectif du spectromètre.

9. Dispositif selon la revendication 1, **caractérisé en ce que**
la distance entre le corps creux (43) et le corps supérieur (5) est réglable.

10. Dispositif selon la revendication 9, **caractérisé en ce que**
le corps creux (43) fait partie intégrante d'une plaque inférieure (1) et **en ce que** le corps supérieur (5) fait partie intégrante d'une plaque supérieure (2), lesdites plaques étant connectées par des montants (3) de longueur réglable.

11. Dispositif selon la revendication 10, **caractérisé en ce que** lesdits montants (3) comprennent une première partie (31) fixée sur la plaque inférieure (1) et une seconde partie (32), vissable sur la première, associée à la plaque supérieure (2), les deuxièmes parties des montants (3) étant contraintes à tourner selon la même quantité.

12. Dispositif selon la revendication 1, **caractérisé en ce que** ledit moyen d'affichage comprend un écran (12).
